# EUROPÄISCHE PATENTSCHRIFT

(11) **EP 1 253 917 B1**
(45) Veröffentlichungstag und Bekanntmachung des Hinweises auf die Patenterteilung: **16.02.2005**
(21) Anmeldenummer: 00985205.4
(22) Anmeldetag: 20.12.2000
(51) Int. Cl.: A61K 31/137, A61K 31/381, A61P 25/00, A61P 9/00, A61P 27/00

(54) **VERWENDUNG VON SUBSTITUIERTEN 4-AMINO-1-PHENYLBUTAN-2-OL-VERBINDUNGEN ALS ARZNEIMITTEL**
USE OF SUBSTITUTED 4-AMINO-1-PHENYLBUTAN-2-OL COMPOUNDS AS MEDICAMENTS
UTILISATION DE COMPOSES 4-AMINO-1-PHENYLBUTAN-2-OL SUBSTITUES EN TANT QUE MEDICAMENTS

(30) Priorität: 27.12.1999 DE 19963175
(43) Veröffentlichungstag der Anmeldung: 06.11.2002
(73) Patentinhaber: Grünenthal GmbH, 52078 Aachen (DE)
(72) Erfinder: SUNDERMANN, Bernd, 52066 Aachen (DE); WNENDT, Stephan, 52062 Aachen (DE); ENGLBERGER, Werner, 52223 Stolberg (DE)
(74) Vertreter: Kutzenberger, Helga, Dr.
(86) Internationale Anmeldenummer: PCT/EP2000/012975
(87) Internationale Veröffentlichungsnummer: WO 2001/047506

(56) Entgegenhaltungen:
- EP-A- 1 043 307
- WO-A-92/05169
- WO-A-99/48492
- IHL R ET AL: "ZUR NOOTROPIKABEWERTUNG FUER DIE PRAXIS" NERVENARZT, SPRINGER VERLAG, BERLIN, DE, Bd. 68, Nr. 11, November 1997 (1997-11), Seiten 853-861, XP001013225 ISSN: 0028-2804
- BERTORELLI R ET AL: "Nociceptin/orphanin FQ and its receptor: a potential target for drug discovery" TRENDS IN PHARMACOLOGICAL SCIENCES, ELSEVIER TRENDS JOURNAL, CAMBRIDGE, GB, Bd. 21, Nr. 7, 1. Juli 2000 (2000-07-01), Seiten 233-234, XP004209778 ISSN: 0165-6147

## Beschreibung

Die Erfindung betrifft die Verwendung substituierter 4-Amino-1-phenylbutan-2-ol-Verbindungen in Form ihrer Racemate, Enantiomeren, Diastereomeren oder entsprechender Basen oder entsprechender Salze von physiologisch verträglichen Säuren als Regulatoren für das Nociceptin/Orphanin-FQ Ligand-ORL1-Rezeptor-System zur Herstellung von Arzneimitteln.

Das Heptadekapeptid Nociceptin/Orphanin FQ ist ein endogener Ligand des ORL1(Opioid-Receptor-Like)-Rezeptors (Meunier et al., Nature 377, 1995, S. 532-535), der zu der Familie der Opioid Rezeptoren gehört und in vielen Regionen des Gehirns und des Rückenmarks zu finden ist (Mollereau et al., FEBS Letters, 341, 1994, S. 33-38, Darland et al., Trends in Neurosciences, 21, 1998, S. 215-221). Das Peptid ist durch eine hohe Affinität, mit einem K_{d}-Wert von annähernd 56 pM (Ardati et al., Mol. Pharmacol. 51, S. 816-824), und durch eine hohe Selektivität für den ORL1-Rezeptor gekennzeichnet. Der ORL1-Rezeptor ist homolog zu den µ, κ und δ Opioid-Rezeptoren und die Aminosäuresequenz des Nociceptin/Orphanin FQ Peptids weist eine starke Ähnlichkeit mit denen der bekannten Opioidpeptide auf. Die durch das Nociceptin/Orphanin FQ induzierte Aktivierung des Rezeptors führt über die Kopplung mit G_{i/o} Proteinen zu einer Inhibierung der Adenylatcyclase (Meunier et al., Nature 377, 1995, S. 532-535). Auch auf der zellulären Ebene sind funktionelle Ähnlichkeiten der µ, κ und δ Opioid-Rezeptoren mit dem ORL1-Rezeptor in Bezug auf die Aktivierung des Kalium-Kanals (Matthes et al., Mol. Pharmacol. 50, 1996, S. 447-450; Vaughan et al., Br. J. Pharmacol. 117, 1996, S. 1609-1611) und der Inhibierung der L-, N- und P/Q-Typ-Kalzium-Kanäle vorhanden (Conner et al., Br. J. Pharmacol. 118, 1996, S. 205-207; Knoflach et al:, J. Neuroscience 16, 1996, S. 6657-6664).

Das Nociceptin/Orphanin-FQ Peptid zeigt nach intercerebroventicularer Applikation eine pronociceptive und hyperalgetische Aktivität in verschiedenen Tiermodellen (Reinscheid et al., Science 270, 1995, S. 792-794; Hara et al,. Br. J. Pharmacol. 121, 1997, S. 401-408). Diese Befunde können als Hemmung der stressinduzierten Analgesie erklärt werden (Mogil et al., Neurosci. Letters 214, 1996, S131-134; sowie Neuroscience 75, 1996, S. 333-337). In diesem Zusammenhang konnte auch eine anxiolytische Aktivität des Nociceptin/Orphanin FQ-Peptides nachgewiesen werden (Jenck et al., Proc. Natl. Acad. Sci. USA 94, 1997, 14854-14858).

Auf der anderen Seite konnte in verschiedenen Tiermodellen, insbesondere nach intrathekaler Applikation, auch ein antinociceptiver Effekt von Nociceptin/Orphanin FQ gezeigt werden. Nociceptin/Orphanin FQ hemmt die Aktivität Kainat- oder Glutamat-stimulierter Hinterwurzelganglienneuronen (Shu et al., Neuropeptides, 32, 1998, 567-571) oder Glutamat-stimulierter Rückenmarksneuronen (Faber et al., Br. J. Pharmacol., 119, 1996, S. 189-190); es wirkt antinociceptiv im Tail Flick-Test in der Maus (King et al., Neurosci. Lett., 223, 1997, 113-116), im Flexor-Reflex-Modell in der Ratte (Xu et al., NeuroReport, 7, 1996, 2092-2094) und im Formalin-Test an der Ratte (Yamamoto et al., Neuroscience, 81, 1997, S. 249-254). In Modellen für neuropathische Schmerzen konnte ebenfalls eine antinociceptive Wirkung von Nociceptin/Orphanin FQ nachgewiesen werden (Yamamoto und Nozaki-Taguchi, Anesthesiology, 87, 1997), die insofern besonders interessant ist, als das die Wirksamkeit von Nociceptin/Orphanin FQ nach Axotomie von Spinalnerven zunimmt. Dies steht im Gegensatz zu den klassischen Opioiden, deren Wirksamkeit unter diesen Bedingungen abnimmt (Abdulla und Smith, J. Neurosci., 18, 1998, S. 9685-9694).

Das Nociceptin/Orphanin FQ-Ligand ORL1-Rezeptor-System ist außerdem noch an der Regulation weiterer physiologischer und pathophysiologischer Prozesse beteiligt. Hierzu gehören unter anderem Lernen und Gedächtnisbildung (Sandin et al., Eur. J. Neurosci., 9, 1997, S. 194-197; Manabe et al., Nature, 394, 1997, S. 577-581 ), Hörvermögen (Nishi et al., EMBO J., 16, 1997, S. 1858-1864), Nahrungsaufnahme (Pomonis et al., NeuroReport, 8, 1996, S. 369-371), Regulation des Blutdruckes (Gumusel et al., Life Sci., 60, 1997, S. 141-145; Campion und Kadowitz, Biochem. Biophys. Res. Comm., 234, 1997, S. 309-312), Epilepsie (Gutierrez et al, Abstract 536.18, Society for Neuroscience, Vol 24, 28th Ann. Meeting, Los Angeles, November 7.-12, 1998) und Diurese (Kapista et al., Life Sciences, 60, 1997, PL 15-21).

Aufgabe der vorliegende Erfindung war es, Arzneimittel zur Verfügung zu stellen, die auf das Nociceptin/Ophanin-FQ-Ligand-ORL1-Rezeptor-System wirken und damit zur Behandlung von Hypotension und/oder Hypertension und/oder seniler Demenz und/oder Alzheimer Erkrankung und/oder allgemeinen kognitiven Dysfunktionen und/oder Tinitus und/oder Schwerhörigkeit und/oder Epilepsie und/oder Fettsucht und/oder Kachexie und/oder zur Anxiolyse und/oder zur Diurese geeignet sind.

Es wurde gefunden, daß substituierte 4-Amino-1-phenylbutan-2-ol-Verbindungen der nachstehenden allgemeinen Formel 1 einen Einfluß auf die Steuerung unterschiedlicher physiologischer und pathophysiologischer Prozesse zeigen, bei denen das Nociceptin/Orphanin-FQ Ligand-ORL1-Rezeptor-System involviert ist. Zu den genannten Prozessen zählen u.a. Angstverhalten, Lernen und Gedächtnisbildung, Regulation des Blutdruckes, Hören, Nahrungsaufnahme, Epilepsie und Diurese.

Gegenstand der vorliegenden Erfindung ist daher die Verwendung wenigstens einer substituierten 4-Amino-1-phenylbutan-2-ol-Verbindung der allgemeinen Formel I, 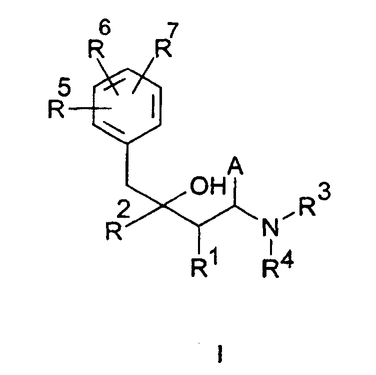 worin
der Rest A einen Aryl- oder Heteroarylrest bedeutet,
die Reste R¹ und R², gleich oder verschieden, für einen C₁₋₆-Alkyl-, vorzugsweise einen C₁₋₃-Alkylrest stehen oder R¹ und R² zusammen einen (CH₂)₂₋₆-Ring bilden, der auch phenylsubstituiert sein kann,
die Reste R³ und R⁴, gleich oder verschieden, für einen C₁₋₆-Alkyl-, vorzugsweise einen C₁₋₃-Alkyl-, einen Aryl-, oder für einen über eine C₁₋₃-Alkylen-Gruppe gebundenen Arylrest stehen oder die Reste R³ und R⁴ zusammen (CH₂)₃₋₆ oder CH₂CH₂OCH₂CH₂ bedeuten,
die Reste R⁵, R⁶, R⁷, gleich oder verschieden, H, F, Cl, Br, l, CF₃, OR⁸, SO₂CH₃, SO₂CF₃, Phenyl, CN, NO₂ oder einen C₁₋₆-Alkylrest bedeuten,
der Rest R⁸ für H, einen C₁₋₆-Alkyl-, vorzugsweise einen C₁₋₃-Alkyl-, einen Aryl-, einen Heteroaryl- oder für einen über eine C₁₋₃-Alkylen-Gruppe gebundenen Aryloder Heteroaryl-Rest steht,
in Form ihres Racemates, ihrer Enantiomeren, ihrer Diastereomeren oder entsprechender Basen oder entsprechender Salze von physiologisch verträglichen Säuren als Regulator für das Nociceptin/Orphanin-FQ Ligand-ORL1-Rezeptor-System.

Unter Alkyl-Resten werden auch verzweigte, unverzweigte und cyclische Kohlenwasserstoffe verstanden, die auch mindestens einfach, vorzugsweise mit einem Halogen- und/oder einem Hydroxyl-Rest, besonders bevorzugt mit Fluor und/oder einem Hydroxyl-Rest substituiert sein können. Enthalten diese mehr als einen Substituenten, so können diese Substituenten gleich oder verschieden sein. Vorzugsweise sind die Alkylreste Methyl, Ethyl, Propyl, 1-Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl, Cyclopropyl, Cyclopropylmethyl, 2-Methylcyclopropyl, Cyclopentyl, Cyclohexyl, CHF₂, CF₃ oder CH₂OH.

Unter einem Arylrest werden auch mindestens einfach mit einem OR⁸-, einem Halogen-, vorzugsweise F- und/oder Cl-, einem CN-, einem NO₂-, einem C₁₋₆-Alkyl- oder einem Phenyl-Rest substituierte Phenyl- oder Naphtyl-Reste verstanden, wobei der Rest R⁸ die oben angegebene Bedeutung hat. Die Phenylreste können auch mit weiteren Ringen kondensiert sein.

Unter einem Heteroarylrest werden auch 5- oder 6-gliedrige, ungesättigte, gegebenenfalls ein System von ankondensierten Arylresten enthaltene, heterocyclische Verbindungen verstanden, die wenigstens ein Heteroatom, vorzugsweise Stickstoff, Sauerstoff und/oder Schwefel, besonders bevorzugt Stickstoff und/oder Sauerstoff enthalten und die ggf. auch mindestens einfach mit einem OR⁸-, einem Halogen-, vorzugsweise F- und/oder Cl-, einem CN-, einem NO₂-, einem C₁₋₆-Alkyl-, oder Phenylrest substituiert sein können, wobei der Rest R⁸ die oben angegebene Bedeutung hat. Vorzugsweise sind die Heteroaryle Furan, Thiophen, Pyrrol, Pyridin, Pyrimidin, Chinolin, Isochinolin, Phthalazin oder Chinazolin.

Bevorzugt sind substituierte 4-Amino-1-phenylbutan-2-ol-Verbindungen der allgemeinen Formel I, in denen R¹ und R² zusammen einen (CH₂)₂₋₆-Ring bilden, der auch phenylsubstituiert sein kann, und R³ bis R⁸ und A die Bedeutung gemäß der allgemeinen Formel I haben.

Ebenfalls bevorzugt sind substituierte 4-Amino-1-phenylbutan-2-ol-Verbindungen der allgemeinen Formel I, in denen A einen unsubstituierten oder wenigstens einfach, vorzugsweise mit OR⁸-, einem F-, Cl-, Br-, einem CN-, einem NO₂-, einem C₁₋₆-Alkyl-, oder einem Phenylrest, substituierten Phenyl-, Thiophenyl- oder Furyl-Rest bedeutet und R¹ bis R⁸ die Bedeutung gemäß der allgemeinen Formel I haben.

Bevorzugt sind auch Verbindungen der allgemeinen Formel I, in denen R⁵ bis R⁷, unabhängig voneinander, H, einen Halogen- oder einen CF₃-Rest bedeutet und R¹ bis R⁴ die Bedeutung gemäß der allgemeinen Formel I haben.

Besonders bevorzugt sind Verbindungen der allgemeinen Formel I, in denen R¹ und R² zusammen einen Cyclohexylring bilden, der auch phenylsubstituiert sein kann, A einen unsubstituierten oder wenigstens einfach, vorzugsweise mit OR⁸-, einem F-, Cl-, Br-, einem CN-, einem NO₂-, einem C₁₋₆-Alkyl-, oder einem Phenylrest, substituierten Phenyl-, Thiophenyl- oder Furyl-Rest bedeutet und R³ bis R⁸ die Bedeutung gemäß der allgemeinen Formel I haben.

Ganz besonders bevorzugt sind die nachfolgenden 4-Amino-1-phenylbutan-2-ol-Verbindungen:
1-(2-Chlorbenzyl)-2-(dimethylaminophenylmethyl)cyclohexanol und das entsprechende Hydrochlorid
1-(3-Chlorbenzyl)-2-(dimethylaminophenylmethyl)cyclohexanol und das entsprechende Hydrochlorid
1-(4-Chlorbenzyl)-2-(dimethylaminophenylmethyl)cyclohexanol und das entsprechende Hydrochlorid
1-(3,4-Difluorbenzyl)-2-(dimethylaminophenylmethyl)cyclohexanol und das entsprechende Hydrochlorid
2-(Dimethylaminophenylmethyl)-1-(3-trifluormethylbenzyl)cyclohexanol und das entsprechende Hydrochlorid
2-(Dimethylaminothiophen-2-ylmethyl)-1-(3-trifluormethylbenzyl)cyclohexanol und das entsprechende Hydrochlorid
2-(Dimethylaminothiophen-3-ylmethyl)-1-(3-trifluormethylbenzyl)cyclohexanol und das entsprechende Hydrochlorid.

Ein weiterer Gegenstand der Erfindung ist die Verwendung wenigstens einer substituierten 4-Amino-1-phenylbutan-2-ol-Verbindung der allgemeinen Formel I als Regulator für das Nociceptin/Orphanin-FQ Ligand-ORL1-Rezeptor-System zur Herstellung eines Arzneimittels zur Behandlung von Hypotension und/oder Hybertension und/oder seniler Demenz und/oder Alzheimer Erkrankung und/oder allgemeinen kognitiven Dysfunktionen und/oder Tinitus und/oder Schwerhörigkeit und/oder Epilepsie und/oder Fettsucht und/oder Kachexie und/oder zur Anxiolyse und/oder zur Diurese.

Ein Gegenstand der Erfindung ist weiterhin auch die Verwendung wenigstens einer substituierten 4-Amino-1-phenylbutan-2-ol-Verbindung der allgemeinen Formel I zur Herstellung eines Arzneimittels zur Behandlung von Hypotension und/oder Hypertension und/oder seniler Demenz und/oder Alzheimer Erkrankung und/oder allgemeinen kognitiven Dysfunktionen und/oder Tinitus und/oder Schwerhörigkeit und/oder Epilepsie und/oder Fettsucht und/oder Kachexie und/oder zur Anxiolyse und/oder zur Diurese.

Zur Zubereitung entsprechender pharmazeutischer Formulierungen werden neben mindestens einer 4-Amino-1-phenylbutan-2-ol-Verbindung der allgemeinen Formel I Trägermaterialien, Füllstoffe, Lösungsmittel, Verdünnungsmittel, Farbstoffe und/oder Bindemittel eingesetzt. Die Auswahl der Hilfsstoffe sowie die einzusetzenden Mengen derselben hängt davon ab, ob das Arzneimittel oral, intravenös, intraperitoneal, intradermal, intramuskulär, intranasal, buccal oder örtlich appliziert werden soll. Für orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Die Verbindungen der allgemeinen Formel I in einem Depot, in gelöster Form oder in einem Pflaster, gegebenenfalls unter Zusatz von die Hautpenetration fördernden Mittel, sind geeignete perkutane Applikationszubereitungen. Oral oder perkutan anwendbare Zubereitungsformen können die Verbindungen der allgemeinen Formel I verzögert freisetzen.

Als physiologisch verträgliche Salze der 4-Amino-1-phenylbutan-2-ol-Verbindungen der allgemeinen Formel I und/oder deren Enantiomeren und/oder deren Diastereomeren können vorzugsweise die Hydrochloride, Hydrobromide, Sulfate, Sulfonate, Phosphate, Tartrate, Embonate, Formiate, Acetate, Propionate, Benzoate, Oxalate, Succinate, Citrate, Glutamate, Fumarate, Aspartate, Glutarate, Stearate, Butyrate, Malonate, Lactate, Mesylate oder ein Gemisch aus wenigstens zwei dieser Salze eingesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,5 bis 50 mg/kg Körpergewicht des Patienten wenigstens einer 4-Amino-1-phenylbutan-2-ol-Verbindung der allgemeinen Formel I appliziert.

Die substituierten 4-Amino-1-phenylbutan-2-ol-Verbindungen der allgemeinen Formel I sind durch die nachfolgenden Verfahren hergestellt worden, wobei die Reste R¹ bis R⁷ sowie A die Bedeutung gemäß der allgemeinen Formel I haben.

Durch die Umsetzung von Mannichbasen der allgemeinen Formel II 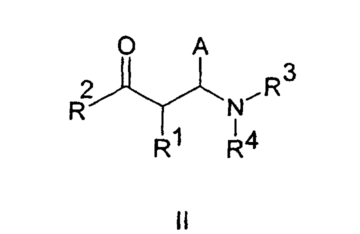 mit substituierten Benzylgrignardverbindungen der allgemeinen Formel III, 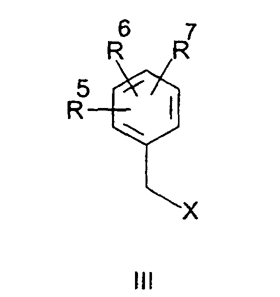 worin X = MgCl, MgBr, Mgl oder Li bedeutet, in einem aliphatischen Ether, vorzugsweise Diethylether und/oder Tetrahydrofuran, einem Kohlenwasserstoff, vorzugsweise Hexan oder Toluol, oder Gemischen aus Kohlenwasserstoffen und aliphatischen Ethern, vorzugsweise bei Temperaturen zwischen -70 °C und +110 °C wurden in Abhängigkeit von den Reaktionsbedingungen bevorzugt tertiäre Alkohole mit der relativen Konfiguration der allgemeinen Formel la erhalten, 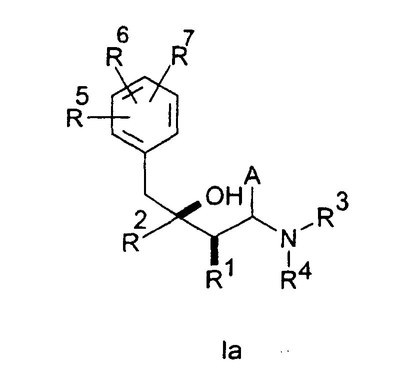 in denen die Aminoarylmethyl- bzw. Aminoheteroarylmethylgruppe *cis* zur Hydroxylgruppe angeordnet ist, wenn R¹ und R² ein Ringsystem bilden. Bei offenkettigen Systemen wird die analoge relative Stereochemie erhalten, die als *anti zu* spezifizieren ist. Die Verbindungen der allgemeinen Formel la lassen sich durch säulenchromatographische Trennung oder durch Kristallisation ihrer Salze, beispielsweise der Hydrochloride, diastereomerenrein erhalten.

Die Mannichbasen der allgemeinen Formel II lassen sich nach literaturbekannten Verfahren (Houben-Weyl - Methoden der Organischen Chemie, E21 b, 1995, S. 1925-1929) durch Umsetzung von Enaminen der allgemeinen Formel IV 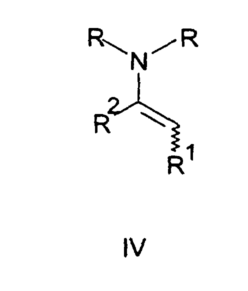 mit einem Imminiumsalz der allgemeinen Formel V, 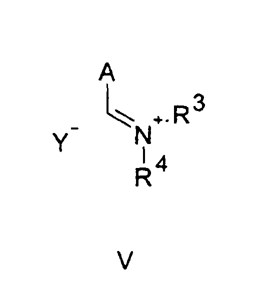 worin Y vorzugsweise Cl⁻, AlCl₄⁻, Br⁻ oder l⁻ bedeutet, erhalten.

Die Enamine der allgemeinen Formel IV werden nach literaturbekannten Verfahren durch die Umsetzung von Ketonen der allgemeinen Formel VI 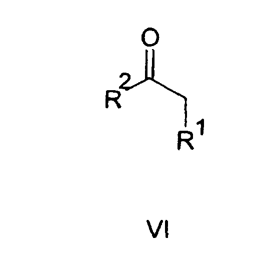 mit sekundären Aminen, vorzugsweise Dimethylamin, Pyrrolidin, Piperidin oder Morpholin, erhalten. (Acta Chem. Scand. B 38, 1984, S. 49-53). Die Imminiumsalze der allgemeinen Formel V werden nach literaturbekannten Verfahren durch Umsetzung von Aminalen der allgemeinen Formel VII 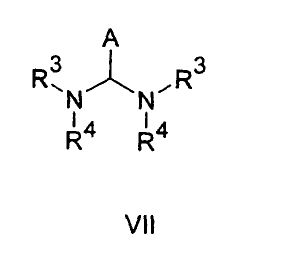 mit Säurechloriden, beispielsweise Acetylchlorid oder Thionylchlorid, hergestellt (Houben-Weyl - Methoden der Organischen Chemie, E21 b, 1995, S. 1925-1929).

Die Imminiumsalze der allgemeinen Formel V müssen dabei nicht isoliert werden, sondern können in situ Erzeugt und mit Enaminen der allgemeinen Formel IV zu Mannichbasen der allgemeinen Formel II umgesetzt werden (Angew. Chem. 106, 1994, S. 2531-2533). Aufgrund der der Keto-Enol-Tautomerie analogen Enamin-Imin-Tautomerie sind statt der Enamine der allgemeinen Formel IV auch Imine der allgemeinen Formel VIII 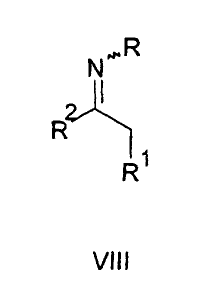 einsetzbar. Alternativ dazu können Ketone der allgemeinen Formel VI auch direkt mit Imminiumsalzen der allgemeinen Formel V umgesetzt werden.

Mannichbasen der allgemeinen Formel II können aber auch durch Umsetzung von Enaminen der allgemeinen Formel IV mit einem aromatischen oder heteroaromatischen Aldehyd der allgemeinen Formel IX 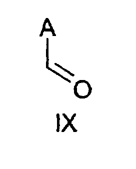 und einem sekundären Amin der allgemeinen Formel HNR³R⁴ (XI) auch in Form des korrespondierenden Hydrochlorids HNR³R⁴·HCl, vorzugsweise in Gegenwart von Triethylamin, Chlortrimethylsilan und Natriumiodid direkt hergestellt werden (Synlett 1997, S. 974-976).

Die Mannichbasen der allgemeinen Formel II werden mit den oben beschriebenen Verfahren in Abhängigkeit von den Reaktionsbedingungen bevorzugt mit der relativen Konfiguration der allgemeinen Formel IIa erhalten, 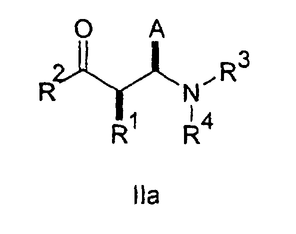 in denen die Aminogruppe *anti* zu R¹ angeordnet ist. Diese Verbindungen der allgemeinen Formel IIa lassen sich durch Kristallisation, auch ihrer Salze, beispielsweise der Hydrochloride, oder durch chromatographische Trennung diastereomerenrein erhalten.

Die Darstellung von Mannichbasen der allgemeinen Formel II durch 1,4-Addition sekundärer Amine der allgemeinen Formel XI an Enone der allgemeinen Formel X, 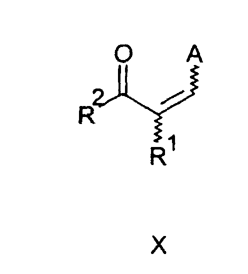 die aus der Aldolkondensation von Ketonen der allgemeinen Formel VI mit aromatischen oder heteroaromatischen Aldehyden der allgemeinen Formel IX erhalten werden, verläuft dagegen weniger stereoselektiv (US-Patent 4,017,637). Diese Vorgehensweise eignet sich daher zur Darstellung der anderen möglichen Stereoisomeren.

Werden chirale Amine zur Darstellung von Enaminen der allgemeinen Formel IV oder Iminen der allgemeinen Formel VIII eingesetzt, so können in der nachfolgenden Mannichreaktion enantiomeren-angereicherte bis enantiomerenreine Mannichbasen der allgemeinen Formel II erhalten werden (Houben-Weyl - Methoden der Organischen Chemie, E21 b, 1995, S. 1925-1929). 4-Amino-1-phenylbutan-2-ol-Verbindungen der allgemeinen Formel I, die ein Phenol enthalten, lassen sich vorzugsweise aus den entsprechenden Methylether-Verbindungen mit Diisobutylaluminiumhydrid in einem aromatischen Kohlenwasserstoff, vorzugsweise Toluol, bei einer Temperatur zwischen 60 und 130 ^{o}C herstellen (Synthesis 1975, S. 617-630).

Die 4-Amino-1-phenylbutan-2-ol-Verbindungen der allgemeinen Formel I wurden in einem Rezeptorbindungsassay mit ³H-Nociceptin/Orphanin FQ mit Membranen von rekombinanten CHO-ORL1 Zellen untersucht. Dieses Testsystem wurde gemäß der von Ardati et al. (Mol. Pharmacol., 51, 1997, S. 816-824) vorgestellten Methode durchgeführt. Die Konzentration von ³H- Nociceptin/Orphanin FQ betrug bei diesen Versuchen 0.5 nM. Die Bindungsassays wurden mit je 20 µg Membranprotein je 200 µl Ansatz in 50 mM Hepes, pH 7,4, 10 mM MgCl₂ und 1 mM EDTA durchgeführt. Die Bindung an den ORL1 -Rezeptor wurde unter Verwendung von je 1 mg WGA-SPA Beads (Amersham-Pharmacia, Freiburg), durch einstündige Inkubation des Ansatzes bei Raumtemperatur und anschliessende Messung im Szintillationscounter Trilux (Wallac, Finnland), bestimmt. Die Affinität wird als Kᵢ-Wert angegeben.

Die folgenden Beispiele dienen der Erläuterung der Erfindung, schränken aber den allgemeinen Erfindungsgedanken nicht ein.

### Beispiele

Die Ausbeuten der hergestellten Verbindungen sind nicht optimiert.

Alle Temperaturen sind unkorrigiert.

Als stationäre Phase für die Säulenchromatographie wurde Kieselgel 60 (0.040-0.063 mm) der Firma E. Merck, Darmstadt eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse der Laufmittel für alle chromatographischen Untersuchungen sind stets in Volumen/Volumen angegeben.

Vol.% bedeutet Volumenprozent und m% bedeutet Massenprozent.

Der Begriff Raumtemperatur bedeutet 20 bis 25 °C.

### Beispiel 1:

### 1-(2-Chlorbenzyl)-2-(dimethylaminophenylmethyl)cyclohexanol, Hydrochlorid

### 1. Stufe

### Benzylidendimethylammoniumchlorid

10 g (56 mmol) N,N,N',N'-Tetramethyl-C-phenylmethandiamin (J. Am. Chem. Soc. 77, 1955, S. 1114-1116) wurden in 100 ml Diethylether gelöst und im Eisbad auf 0°C gekühlt. Es wurden unter Stickstoff 4,0 ml (56 mmol) Acetylchlorid zugetropft. Nach den ersten Tropfen fiel ein weißes Salz aus, die Temperatur erhöhte sich leicht. Nach 15 Stunden bei Raumtemperatur wurde abdekantiert, der Feststoff dreimal mit je 100 ml Diethylether gewaschen, über eine Schutzgasfritte unter Stickstoff filtriert und im Ölpumpenvakuum bis zur Gewichtskonstanz getrocknet. Auf diese Weise wurden 7,7 g Benzylidendimethylammoniumchlorid (entsprechend 81 % der theoretisch berechneten Ausbeute) erhalten.

### 2. Stufe

### 2-(Dimethylaminophenylmethyl)cyclohexanon

7,1 ml (44 mmol) 1-(Pyrrolidino)-1-cyclohexen wurden in 45 ml Dichlormethan gelöst und unter Stickstoff mit einem Trockeneis/Isopropanol-Bad auf -70 °C gekühlt. Unter Rühren wurden 7,5 g (44 mmol) Benzylidendimethylammoniumchlorid aus Stufe 1 zugegeben, die Mischung innerhalb von zwei bis drei Stunden auf -30°C erwärmt und 15 Stunden bei dieser Temperatur gelagert.
Zur Aufarbeitung wurden 60 ml halbkonzentrierte Salzsäure zugegeben und 5 Minuten nachgerührt. Bei Raumtemperatur wurde mit 50 ml Diethylether gewaschen, die wässrige Phase mit 440 ml Ammoniaklösung (25 Vol.%) versetzt und schnell dreimal mit je 150 ml Diethylether extrahiert. Die vereinigten organischen Extrakte wurden über Natriumsulfat getrocknet, filtriert und am Rotationsverdampfer ohne Wärmezufuhr (500 bis 10 mbar) eingeengt. Auf diese Weise wurden 10,1 g Rohbase (entsprechend 99,5 % der theoretisch berechneten Ausbeute) erhalten.
9,81 g (42,4 mmol) der Rohbase wurden in 83 ml 2-Butanon gelöst und nacheinander 0,76 ml (42,2 mmol) Wasser und 5,36 ml (42,4 mmol) Chlortrimethylsilan zugegeben. Der Ansatz wurde 15 Stunden bei Raumtemperatur aufbewahrt, der ausgefallene Feststoff abgesaugt, mit kleinen Portionen Diethylether gewaschen und im Ölpumpenvakuum bis zur Gewichtskonstanz getrocknet. Auf diese Weise wurden 8,92 g des Hydrochlorids von 2-(Dimethylaminophenylmethyl)cyclohexanon (entsprechend 79 % der theoretisch berechneten Ausbeute) erhalten.

### 3. Stufe

### 1-(2-Chlorbenzyl)-2-(dimethylaminophenylmethyl)cyclohexanol, Hydrochlorid

0,38 g (15,6 mmol) Magnesiumspäne wurden in 15 ml Diethylether p.a. gerührt. Es wurden 2,0 ml (15,6 mmol) 2-Chlorbenzylchlorid, gelöst in 15 ml Diethylether, so zugetropft, daß das Reaktionsgemisch leicht siedete. Nach der Zugabe wurde eine Stunde bei Raumtemperatur nachgerührt. 3,0 g (13,0 mmol) des nach Stufe 2 hergestellten 2-(Dimethylaminophenylmethyl)cyclohexanons wurden in 15 ml Diethylether gelöst, bei Eisbadkühlung zum Grignard-Ansatz getropft und 15 Stunden bei Raumtemperatur gerührt.
Zur Aufarbeitung wurden 30 ml gesättigte Ammoniumchloridlösung bei Eisbadkühlung zugegeben und bei Raumtemperatur dreimal mit je 60 ml Essigsäureethylester extrahiert. Die vereinigten organischen Extrakte wurden über Natriumsulfat getrocknet, filtriert und am Rotationsverdampfer (500 bis 10 mbar) eingeengt. Es wurden 4,50 g Rohbase (entsprechend 97 % der theoretisch berechneten Ausbeute) erhalten, aus der nach Beispiel 1 (2. Stufe) mit Chlortrimethylsilan/Wasser in 2-Butanon ein Hydrochlorid gefällt wurde. Hieraus wurde mit 40 ml Wasser und 5 ml Natronlauge (32 m%) die Base freigesetzt, dreimal mit je 40 ml Diethylether extrahiert, die vereinigten organischen Extrakte über Natriumsulfat getrocknet, filtriert und am Rotationsverdampfer (500 bis 10 mbar) eingeengt. Man erhielt 2,90 g Rohbase, die auf eine 3,5 x 15 cm Säule, gefüllt mit Kieselgel, gegeben wurden. Die Elution mit Essigsäureethylester/n-Hexan 2:5 ergab 1,59 g Base, aus der nach Beispiel 1 (2. Stufe) mit Chlortrimethylsilan/Wasser in 2-Butanon 1,75 g 1-(2-Chlorbenzyl)-2-(dimethylaminophenylmethyl)cyclohexanol, Hydrochlorid (entsprechend 34 % der theoretisch berechneten Ausbeute) erhalten, das sich beim Erhitzen ab 130 °C zersetzt.

### Beispiel 2:

### 1-(3-Chlorbenzyl)-2-(dimethylaminophenylmethyl)cyclohexanol, Hydrochlorid

0,38 g (15,6 mmol) Magnesiumspäne wurden in 15 ml Diethylether p.a. gerührt. Es wurden 2,0 ml (15,6 mmol) 3-Chlorbenzylchlorid, gelöst in 15 ml Diethylether, so zugetropft, daß das Reaktionsgemisch leicht siedete. Nach der Zugabe wurde eine Stunde bei Raumtemperatur nachgerührt. 3,0 g (13,0 mmol) des nach Beispiel 1 hergestellten 2-(Dimethylaminophenylmethyl)cyclohexanons wurden in 15 ml Diethylether gelöst, bei Eisbadkühlung zum Grignard-Ansatz getropft und 15 Stunden bei Raumtemperatur gerührt.
Zur Aufarbeitung wurden 30 ml gesättigte Ammoniumchloridlösung bei Eisbadkühlung zugegeben und bei Raumtemperatur dreimal mit je 60 ml Essigsäureethylester extrahiert. Die vereinigten organischen Extrakte wurden über Natriumsulfat getrocknet, filtriert und am Rotationsverdampfer (500 bis 10 mbar) eingeengt. Es wurden 4,55 g Rohbase (entsprechend 98 % der theoretisch berechneten Ausbeute) erhalten, aus der nach Beispiel 1 (2. Stufe) mit Chlortrimethylsilan/Wasser in 2-Butanon ein Hydrochlorid gefällt wurde. Hieraus wurde mit 40 ml Wasser und 5 ml Natronlauge (32 m%) die Base freigesetzt, dreimal mit je 40 ml Diethylether extrahiert, die vereinigten organischen Extrakte über Natriumsulfat getrocknet, filtriert und am Rotationsverdampfer (500 bis 10 mbar) eingeengt.
Man erhielt 2,87 g Rohbase, die mit 5 ml Essigsäureethylester/n-Hexan 2:5 versetzt wurden. Der unlösliche Rückstand wurde abfiltriert und getrocknet. Man erhielt 2,11 g Base, aus der nach Beispiel 1 (2. Stufe) mit Chlortrimethylsilan/Wasser in 2-Butanon 1,68 g 1-(3-Chlorbenzyl)-2-(dimethylaminophenylmethyl)cyclohexanol, Hydrochlorid (entsprechend 33 % der theoretisch berechneten Ausbeute) mit einem Schmelzpunkt von 185 - 188 °C gefällt wurde.

### Beispiel 3:

### 1-(4-Chlorbenzyl)-2-(dimethylaminophenylmethyl)-1-cyclohexanol, Hydrochlorid

0,38 g (15,6 mmol) Magnesiumspäne wurden in 15 ml Diethylether p.a. gerührt. Es wurden 1,99 g (15,6 mmol) 4-Chlorbenzylbromid, gelöst in 15 ml Diethylether, so zugetropft, daß das Reaktionsgemisch leicht siedete. Nach der Zugabe wurde eine Stunde bei Raumtemperatur nachgerührt. 3,0 g (13,0 mmol) des nach Beispiel 1 hergestellten 2-(Dimethylaminophenylmethyl)cyclohexanons wurden in 15 ml Diethylether gelöst, bei Eisbadkühlung zum Grignard-Ansatz getropft und 15 Stunden bei Raumtemperatur gerührt.
Zur Aufarbeitung wurden 30 ml gesättigte Ammoniumchloridlösung bei
Eisbadkühlung zugegeben und bei Raumtemperatur dreimal mit je 60 ml Essigsäureethylester extrahiert. Die vereinigten organischen Extrakte wurden über Natriumsulfat getrocknet, filtriert und am Rotationsverdampfer (500 bis 10 mbar) eingeengt. Es wurden 4,48 g Rohbase (entsprechend 97 % der theoretisch berechneten Ausbeute) erhalten.
Aus der Rohbase wurden nach Beispiel 1 (2. Stufe) mit Chlortrimethylsilan/Wasser in 2-Butanon 1,74 g 1-(4-Chlorbenzyl)-2-(dimethylaminophenylmethyl)-1-cyclohexanol, Hydrochlorid (entsprechend 34 % der theoretisch berechneten Ausbeute) erhalten, das sich beim Erhitzen ab 208°C zersetzt.

### Beispiel 4:

### 1-(3,4-Difluorbenzyl)-2-(dimethylaminophenylmethyl)cyclohexanol, Hydrochlorid

0,29 g (11,9 mmol) Magnesiumspäne wurden in 5 ml Diethylether p.a. gerührt. Es wurden 2,47 g (11,9 mmol) 3,4-Difluorbenzylbromid, gelöst in 10 ml Diethylether, so zugetropft, daß das Reaktionsgemisch leicht siedete. Nach der Zugabe wurde eine Stunde bei Raumtemperatur nachgerührt. 2,30 g (9,9 mmol) des nach Beispiel 1 hergestellten 2-(Dimethylaminophenylmethyl)cyclohexanons wurden in 10 ml Diethylether gelöst, bei Eisbadkühlung zum Grignard-Ansatz getropft und 15 Stunden bei Raumtemperatur gerührt.

Zur Aufarbeitung wurden 15 ml gesättigte Ammoniumchloridlösung bei Eisbadkühlung zugegeben und bei Raumtemperatur dreimal mit je 15 ml Diethylether extrahiert. Die vereinigten organischen Extrakte wurden über Natriumsulfat getrocknet, filtriert und am Rotationsverdampfer (500 bis 10 mbar) eingeengt. Es wurden 3,53 g Rohbase (entsprechend 100 % der theoretisch berechneten Ausbeute) erhalten, aus der nach Beispiel 1 (2. Stufe) mit Chlortrimethylsitan/Wasser in 2-Butanon ein Hydrochlorid gefällt wurde. Hieraus wurde mit 30 ml Wasser und 10 ml Ammoniaklösung (25 Vol.%) die Base freigesetzt, dreimal mit je 20 ml Diethylether extrahiert, die vereinigten organischen Extrakte über Natriumsulfat getrocknet, filtriert und am Rotationsverdampfer (500 bis 10 mbar) eingeengt. Es wurden 2,31 g Rohbase (65 % der Theorie) erhalten, aus der nach Beispiel 1 (2. Stufe) mit Chlortrimethylsilan/Wasser in 2-Butanon 2,0 g 1-(3,4-Difluorbenzyl)-2-(dimethylaminophenylmethyl)cyclohexanol, Hydrochlorid (entsprechend 51 % der theoretisch berechneten Ausbeute) mit einem Schmelzpunkt von 185 - 188 °C gefällt wurden.

### Beispiel 5:

### 1. Stufe: 2-(Dimethylaminophenylmethyl)cyclohexanon

Zu 620 ml (620 mmol) Natriumiodidlösung (1 M in Acetonitril), mit einem Eisbad auf 0 °C gekühlt, wurden unter Rühren 32,1 g (283 mmol) frisch getrocknetes Dimethylamin-Hydrochlorid gegeben, 79 ml (565 mmol) Triethylamin und 79 ml (620 mmol) Chlortrimethylsilan zugetropft und eine Stunde bei Raumtemperatur nachgerührt. Bei Eiskühlung wurden 30,0 g (283 mmol) Benzaldehyd zugegeben und noch eine Stunde bei Raumtemperatur weitergerührt. Es wurde erneut mit einem Eisbad auf 0 °C gekühlt, 42,7 g (283 mmol) 1-(Pyrrolidino)-1-cyclohexen zugegeben und zwei Stunden bei Raumtemperatur weitergerührt.
Zur Aufarbeitung wurde der Ansatz bei Eiskühlung mit 420 ml halbkonzentrierter Salzsäure versetzt, 10 Minuten gerührt, zweimal mit je 420 ml Diethylether gewaschen und mit 1060 ml verdünnter Ammoniaklösung (5 Vol.%) alkalisch gestellt (pH ca. 9). Es wurde dreimal mit je 420 ml Diethylether extrahiert, die vereinigten organischen Extrakte über Natriumsulfat getrocknet, filtriert und am Rotationsverdampfer (500 bis 10 mbar) ohne Wärmezufuhr eingeengt. Es wurden 54,1 g 2-(Dimethylaminophenylmethyl)cyclohexanon (entsprechend 83 % der theoretisch berechneten Ausbeute) erhalten.

### 2. Stufe: 2-(Dimethylaminophenylmethyl)-1-(3-trifluormethylbenzyl)cyclohexanol, Hydrochlorid

0,38 g (15,6 mmol) Magnesiumspäne wurden in 10 ml Diethylether p.a. gerührt. Es wurden 3,03 g (15,6 mmol) 3-Chlormethylbenzotrifluorid, gelöst in 10 ml Diethylether, so zugetropft, daß das Reaktionsgemisch leicht siedete. Nach der Zugabe wurde eine Stunde bei Raumtemperatur nachgerührt. 3,00 g (13,0 mmol) des nach Stufe 1 hergestellten 2-(Dimethylaminophenylmethyl)cyclohexanons wurden in 15 ml Diethylether gelöst, bei Eisbadkühlung zum Grignard-Ansatz getropft und 15 Stunden bei Raumtemperatur gerührt.
Zur Aufarbeitung wurden 20 ml gesättigte Ammoniumchloridlösung bei Eisbadkühlung zugegeben und bei Raumtemperatur dreimal mit je 20 ml Diethylether extrahiert. Die vereinigten organischen Extrakte wurden über Natriumsulfat getrocknet, filtriert und am Rotationsverdampfer (500 bis 10 mbar) eingeengt. Es wurden 4,90 g Rohbase (entsprechend 96 % der theoretisch berechneten Ausbeute) erhalten, aus der nach Beispiel 1 (2. Stufe) mit Chlortrimethylsilan/Wasser in 2-Butanon 3,16 g 2-(Dimethylaminophenylmethyl)-1-(3-tri-fluormethylbenzyl)cyclohexanol, Hydrochlorid (entsprechend 57 % der theoretisch berechneten Ausbeute) mit einem Schmelzpunkt von 184 - 186 °C erhielt.

### Beispiel 6:

### 1. Stufe: [2-Dimethylaminothiophen-2-ylmethyl]cyclohexanon

Zu 118 ml (118 mmol) Natriumiodidlösung (1 M in Acetonitril), mit einem Eisbad auf 0°C gekühlt, wurden unter Rühren 4,36 g (53,5 mmol) frisch getrocknetes Dimethylamin-Hydrochlorid gegeben, 15 ml (107 mmol) Triethylamin und 15 ml (118 mmol) Chlortrimethylsilan zugetropft und eine Stunde bei Raumtemperatur nachgerührt. Bei Eiskühlung wurden 6,0 g (53,5 mmol) Thiophen-2-carboxaldehyd zugegeben und noch eine Stunde bei Raumtemperatur weitergerührt. Es wurde erneut mit einem Eisbad auf 0°C gekühlt, 8,6 ml (53.5 mmol) 1-(Pyrrolidino)-1-cyclohexen zugegeben und zwei Stunden bei Raumtemperatur weitergerührt. Zur Aufarbeitung wurde der Ansatz bei Eiskühlung mit 80 ml halbkonzentrierter Salzsäure versetzt, 10 Minuten gerührt, zweimal mit je 80 ml Ether gewaschen und mit 200 ml verdünnter Ammoniaklösung (5 Vol-%) alkalisch gestellt (pH ca. 9). Es wurde dreimal mit je 80 ml Ether extrahiert, die vereinigten organischen Extrakte über Natriumsulfat getrocknet, filtriert und am Rotationsverdampfer (500 bis 10 mbar) eingeengt. Es wurden 8,09 g Rohbase von 2-(Dimethylaminothiophen-2-ylmethyl)cyclohexanon (63,7 % der Theorie) erhalten.

### 2. Stufe

### 2-(Dimethylaminothiophen-2-ylmethyl)-1-(3-trifluormethylbenzyl)cyclohexanol, Hydrochlorid

0,31 g (12,6 mmol) Magnesiumspäne wurden in 10 ml Ether p.a. gerührt. Es wurden 2,46 g (12,6 mmol) 3-Chlormethylbenzotrifluorid, gelöst in 10 ml Ether, so zugetropft, daß das Reaktionsgemisch leicht siedete. Nach beendeter Zugabe wurde eine Stunde bei Raumtemperatur nachgerührt. 2,50 g (10,5 mmol) des nach Stufe 1 hergestellten 2-(Dimethylaminothiophen-2-ylmethyl)cyclohexanons wurden in 10 ml Ether gelöst, bei Eisbadkühlung zum Grignard-Ansatz getropft und 15 Stunden bei Raumtemperatur gerührt. Zur Aufarbeitung wurden 15 ml gesättigte Ammoniumchloridlösung bei Eisbadkühlung zugegeben und bei Raumtemperatur dreimal mit je 20 ml Ether extrahiert. Die vereinigten organischen Extrakte wurden über Natriumsulfat getrocknet, filtriert und am Rotationsverdampfer (500 bis 10 mbar) eingeengt. Es wurden 3,33 g Rohbase (entsprechend 79,6 % der theoretisch berechneten Ausbeute) erhalten, aus der nach Beispiel 1 (3. Stufe) mit Chlortrimethylsilan/Wasser in 2-Butanon ein Hydrochlorid gefällt wurde. Hieraus wurde mit 20 ml Wasser und 5 ml Ammoniaklösung (25 Vol-%) die Base freigesetzt, dreimal mit je 20 ml Ether extrahiert, die vereinigten organischen Extrakte über Natriumsulfat getrocknet, filtriert und am Rotationsverdampfer (500 bis 10 mbar) eingeengt. Es wurden 0,63 g Rohbase (15,0 % der Theorie) erhalten, aus der nach Beispiel 1 (2. Stufe) mit Chlortrimethylsilan/Wasser in 2-Butanon 0,39 g 2-[Dimethylamino-thiophen-2-ylmethyl]-1-(3-trifluormethylbenzyl)cyclohexanol, Hydrochlorid (8,5 % der Theorie erhielt, das sich ab 98 °C zersetzt.

### Beispiel 7:

### 2-(Dimethylaminothiophen-3-ylmethyl)-1-(3-trifluormethylbenzyl)cyclohexanol, Hydrochlorid

Wie für Beispiel 6 beschrieben wurde zunächst 2-(Dimethylaminothiophen-3-ylmethyl)cyclohexanon aus Dimethylamin-Hydrochlorid, Thiophen-3-carbaldehyd und 1-(Pyrrolidino)-1-cyclohexen hergestellt und anschließend durch Umsetzung mit 3-Chlormethylbenzotrifluorid in einer Grignardreaktion und abschließende Hydrochloridfällung 2-(Dimethylaminothiophen-3-ylmethyl)-1-(3-trifluormethylbenzyl)cyclohexanol, Hydrochlorid erhalten.

### Molekularpharmakologische Untersuchungen:

Von jeder dieser Verbindungen der Beispiele 1 bis 7 wurde gemäß den angegebenen molekutarpharmakologischen Untersuchungen die Affinität zum ORL1-Rezeptor bestimmt. Die entsprechenden Kᵢ-Werte sind in der nachfolgenden Tabelle 1 angegeben.

**Tabelle 1:**

| Beispiel | Kᵢ (µM) ORL1-Bindungsassay |
|---|---|
| 1 | 6,0 |
| 2 | 2,1 |
| 3 | 1,7 |
| 4 | 1,9 |
| 5 | 0,90 |
| 6 | 0,60 |
| 7 | 0,33 |

## Patentansprüche

1. Verwendung von wenigstens einer substituierten 4-Amino-1-phenylbutan-2-ol-Verbindung der allgemeinen Formel I, 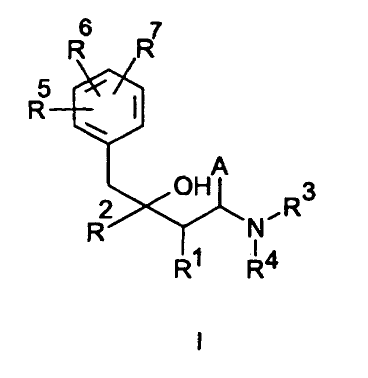 worin
der Rest A einen Aryl- oder Heteroarylrest bedeutet,
die Reste R¹ und R², gleich oder verschieden, für einen C₁₋₆-Alkyl-, vorzugsweise einen C₁₋₃-Alkylrest stehen oder R¹ und R² zusammen einen (CH₂)₂₋₆-Ring bilden, der auch phenylsubstituiert sein kann,
die Reste R³ und R⁴, gleich oder verschieden, für einen C₁₋₆-Alkyl-, vorzugsweise einen C₁₋₃-Alkyl-, einen Aryl-, oder für einen über eine C₁₋₃-Alkylen-Gruppe gebundenen Arylrest stehen oder R³ und R⁴ zusammen (CH₂)₃₋₆ oder CH₂CH₂OCH₂CH₂ bedeuten,
die Reste R⁵, R⁶, R⁷, gleich oder verschieden, H, F, Cl, Br, l, CF₃, OR⁸, SO₂CH₃, SO₂CF₃, Phenyl, CN, NO₂ oder einen C₁₋₆-Alkylrest bedeuten,
der Rest R⁸ für H, einen C₁₋₆-Alkyl-, vorzugsweise einen C₁₋₃-Alkyl-, einen Aryl-, einen Heteroaryl- oder für einen über eine C₁₋₃-Alkylen-Gruppe gebundenen Aryl- oder Heteroaryl-Rest-steht;
in Form ihres Racemates, ihrer Enantiomeren, ihrer Diastereomeren oder entsprechender Basen oder entsprechender Salze von physiologisch verträglichen Säuren als Regulator für den ORL1 (Opioid Receptor Like) Rezeptor des Nociceptin/Orphanin-FQ-Ligand-ORL1-Rezeptor-Systemes zur Herstellung eines Arzneimittels zur Anxiolyse oder zur Diurese oder zur Behandlung von Hypotension oder Hypertension oder seniler Demenz oder Alzheimer Erkrankung oder allgemeinen kognitiven Dysfunktionen oder Tinitus oder Schwerhörigkeit oder Epilepsie oder Fettsucht oder Kachexie.

2. Verwendung nach Anspruch 1 **dadurch gekennzeichnet, daß** R¹ und R2 zusammen einen (CH₂)₂₋₆-Ring bilden, der auch phenylsubstituiert sein kann, und R³ bis R⁸ und A die Bedeutung gemäß Anspruch 1 haben.

3. Verwendung nach Anspruch 1, **dadurch gekennzeichnet, daß** A einen unsubstituierten oder wenigstens einfach, vorzugsweise mit OR⁸-, einem F-, Cl-, Br-, einem CN-, einem NO₂-, einem C₁₋₆-Alkyl-, oder einem Phenylrest, substituierten Phenyl-, Thiophenyl- oder Furyl-Rest bedeutet und R¹ bis R⁸ die Bedeutung gemäß Anspruch 1 haben.

4. Verwendung nach Anspruch 1, **dadurch gekennzeichnet, daß** R⁵ bis R⁷, gleich oder verschieden, H einen Halogen- oder einen CF₃-Rest bedeuten und R¹ bis R⁴ die Bedeutung gemäß Anspruch 1 haben.

5. Verwendung nach Anspruch 1, **dadurch gekennzeichnet, daß** R¹ und R2 zusammen einen Cyclohexylring bilden, der auch phenylsubstituiert sein kann, A einen unsubstituierten oder wenigstens einfach, vorzugsweise mit OR⁸-, einem F-, Cl-, Br-, einem CN-, einem NO₂-, einem C₁₋₆-Alkyl-, oder einem Phenylrest, substituierten Phenyl-, Thiophenyl oder Furyl bedeutet und R3 bis R⁸ die Bedeutung gemäß Anspruch 1 haben.

6. Verwendung nach Anspruch 1, **dadurch gekennzeichnet, daß** als substituierte 4-Amino-1-phenyibutan-2-ol-Verbindungen
1-(2-Chlorbenzyl)-2-(dimethylaminophenylmethyl)cyclohexanol und/oder das entsprechende Hydrochlorid
1-(3-Chlorbenzyl)2-(dimethylaminophenylmethyl)cyclohexanol und/oder das entsprechende Hydrochlorid
1-(4-Chlorbenzyl)-2-(dimethylaminophenylmethyl)cyclohexanol und/oder das entsprechende Hydrochlorid
1-(3,4-Difluorbenzyl)-2-(dimethylaminophenylmethyl)cyclohexanol und/oder das entsprechende Hydrochlorid
2-(Dimethylaminothiophen-2-ylmethyl-1-(3-thrifluormethylbenzyl)cyclohexanol und das entsprechende Hydrochlorid
2-(Dimethylaminothiophen-3-ylmethyl)-1-(3-trifluormethylbenzyl)cyclohexanol und das entsprechende Hydrochlorid
und/oder
2-(Dimethylaminophenylmethyl)-1-(3-trifluormethylbenzyl)cyclohexanol und/oder das entsprechende Hydrochlorid eingesetzt werden.

## Claims

1. Use of at least substituted 4-amino-1-phenylbutan-2-ol compound of the general formula I 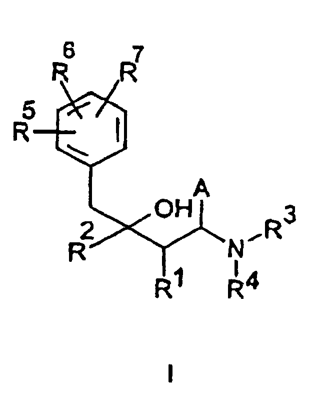 wherein
the radical A represents an aryl or heteroaryl radical,
the radicals R¹ and R², which may be identical or different, represent a C₁₋₆-alkyl radical, preferably a C₁₋₃-alkyl radical, or R¹ and R² together form a (CH₂)₂₋₆ ring, which may also be substituted by phenyl,
the radicals R³ and R⁴, which may be identical or different, represent a C₁₋₆-alkyl radical, preferably a C₁₋₃-alkyl radical, an aryl radical, or an aryl radical bonded *via* a C₁₋₃-alkylene group, or R³ and R⁴ together represent (CH₂)₃₋₆ or CH₂CH₂OCH₂CH₂,
the radicals R⁵, R⁶ and R⁷, which may be identical or different, represent H, F, Cl, Br, I, CF₃, OR⁸, SO₂CH₃, SO₂CF₃, phenyl, CN, NO₂ or a C₁₋₆-alkyl radical,
the radical R⁸ represents H, a C₁₋₆-alkyl radical, preferably a C₁₋₃-alkyl radical, an aryl radical, a heteroaryl radical, or an aryl or heteroaryl radical bonded *via* a C₁₋₃-alkylene group,
in the form of its racemate, its enantiomers, its diastereoisomers or corresponding bases or corresponding salts of physiologically tolerable acids, as a regulator for the ORL1 (opioid-receptor-like) receptor of the nociceptin/orphanin FQ ligand-ORL1 receptor system in the preparation of a medicament for anxiolysis or for diuresis or for the treatment of hypotension or hypertension, or senile dementia or Alzheimer's disease or general cognitive dysfunctions or tinnitus or hardness of hearing or epilepsy or obesity or cachexia.

2. Use according to claim 1, **characterised in that** R¹ and R² together form a (CH₂)₂₋₆ ring, which may also be substituted by phenyl, and R³ to R⁸ and A are as defined according to claim 1,

3. Use according to claim 1, **characterised in that** A represents a phenyl, thiophenyl or furyl radical that is unsubstituted or is substituted at least once, preferably by OR⁸, an F, Cl, Br radical, a CN radical, an NO₂ radical, a C₁₋₆-alkyl radical or by a phenyl radical, and R¹ to R⁸ are as defined according to claim 1.

4. Use according to claim 1, **characterised in that** R⁵ to R⁷, which may be identical or different, represent H, a halogen radical or a CF₃ radical, and R¹ to R⁴ are as defined according to claim 1.

5. Use according to claim 1, **characterised in that** R¹ and R² together form a cyclohexyl ring, which may also be substituted by phenyl, A represents a phenyl, thiophenyl or furyl radical that is unsubstituted or is substituted at least once, preferably by OR⁸, an F, Cl, Br radical, a CN radical, an NO₂ radical, a C₁₋₆-alkyl radical or by a phenyl radical, and R³ to R⁸ are as defined according to claim 1.

6. Use according to claim 1, **characterised in that** there are used as substituted 4-amino-1-phenylbutan-2-ol compounds
1-(2-chlorobenzyl)-2-(dimethylaminophenylmethyl)cyclohexanol and/or the corresponding hydrochloride
1-(3-chlorobenzyl)-2-(dimethylaminophenylmethyl)cyclohexanol and/or the corresponding hydrochloride
1-(4-chlorobenzyl)-2-(dimethylaminophenylmethyl)cyclohexanol and/or the corresponding hydrochloride
1-(3,4-difluorobenzyl)-2-(dimethylaminophenylmethyl)cyclohexanol and/or the corresponding hydrochloride
2-(dimethylaminothiophen-2-ylmethyl)-1-(3-trifluoromethylbenzyl)cyclohexanol and the corresponding hydrochloride
2-(dimethylaminothiophen-3-ylmethyl)-1-(3-trifluoromethylbenzyl)cyclohexanol and the corresponding hydrochloride
and/ or
2-(dimethylaminophenylmethyl)-1-(3-trifluoromethylbenzyl)cyclohexanol and/or the corresponding hydrochloride.

## Revendications

1. Utilisation d'au moins un composé 4-amino-1-phénylbutan-2-ol substitué de formule générale I, 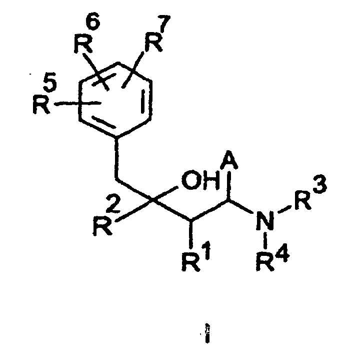 dans laquelle
le reste A représente un radical aryle ou hétéroaryle,
les restes R¹ et R², identiques ou différents, représentent un radical alkyle en C₁-C₆, de préférence un radical alkyle en C₁-C₃, ou R¹ et R² forment ensemble un cycle (CH₂)₂₋₆ qui peut également être substitué par un groupe phényle,
les reste R³ et R⁴, identiques ou différents, représentent un radical alkyle en C₁-C₆, de préférence un radical alkyle en C₁-C₃, un radical aryle ou un radical aryle relié par un groupe alkylène en C₁-C₃, ou R³ et R⁴ représentent ensemble (CH₂)₃₋₆ ou CH₂CH₂OCH₂CH₂,
les restes R⁵, R⁶, R⁷, identiques ou différents, représentent H, F, Cl, Br., I, CF₃, OR⁸, SO₂CH₃, SO₂CF₃, phényle, CN, NO₂ ou un radical alkyle en C₁-C₆,
le radical R⁸ représente H, un radical alkyle en C₁-C₆, de préférence un radical alkyle en C₁-C₃, un radical aryle, un radical hétéroaryle ou un radical aryle ou hétéroaryle relié par un groupe alkylène en C₁-C₃,
sous forme de son racémique, de ses énantiomères, de ses diastéréoisomères ou de bases correspondantes ou de sels correspondants avec des acides physiologiquement acceptables, en tant que régulateur de récepteur ORL1 (ressemblant au récepteur d'opioïde) du système récepteur de nociceptine/orphanine-ligand-FQ-ligand-ORL1, pour la fabrication d'un médicament destiné à la réduction de l'anxiété ou à la diurèse ou au traitement de l'hypotension ou de l'hypertension ou de la démence sénile ou de la maladie d'Alzheimer ou de dysfonctionnements cognitifs généraux ou de l'acouphène ou de la surdité ou de l'épilepsie ou de l'obésité ou de la cachexie.

2. Utilisation selon la revendication 1, **caractérisée en ce que** R¹ et R² forment ensemble un cycle (CH₂)₂₋₆ qui peut également être substitué par un groupe phényle, et R³ à R⁸ et A ont les significations selon la revendication 1.

3. Utilisation selon la revendication 1, **caractérisée en ce que** A représente un radical phényle, thiophényle ou furyle non substitué ou substitué au moins une fois, de préférence par OR⁸, par un atome de F, Cl, Br ou par un radical CN, NO₂, alkyle en C₁-C₆ ou phényle, et R¹ à R⁸ ont les significations selon la revendication 1.

4. Utilisation selon la revendication 1, **caractérisée en ce que** R⁵ à R⁷, identiques ou différents, représentent un atome d'hydrogène ou d'halogène ou un radical CF₃, et R¹ à R⁴ ont les significations selon la revendication 1.

5. Utilisation selon la revendication 1, **caractérisée en ce que** R¹ et R² forment ensemble un cycle cyclohexyle qui peut également être substitué par un groupe phényle, A représente un radical phényle, thiophényle ou furyle non substitué ou substitué au moins une fois, de préférence par OR⁸, par un atome de F, Cl, Br ou par un radical CN, NO₂, alkyle en C₁-C₆ ou phényle, et R³ à R⁸ ont les significations selon la revendication 1.

6. Utilisation selon la revendication 1, **caractérisée en ce qu'**on utilise en tant que composés 4-amino-1-phénylbutan-1-ol substitués
le 1-(2-chlorobenzyl)-2-(diméthylaminophénylméthyl)-cyclohexanol et/ou le chlorhydrate correspondant
le 1-(3-chlorobenzyl)-2-(diméthylaminophénylméthyl)-cyclohexanol et/ou le chlorhydrate correspondant
le 1-(4-chlorobenzyl)-2-(diméthylaminophénylméthyl)-cyclohexanol et/ou le chlorhydrate correspondant
le 1-(3,4-difluorobenzyl)-2-(diméthylaminophénylméthyl)-cyclohexanol et/ou le chlorhydrate correspondant
le 2-(diméthylaminothiophén-2-ylméthyl)-1-(3-trifluorométhylbenzyl) cyclohexanol et le chlorhydrate correspondant
le 2-(diméthylaminothiophén-3-ylméthyl)-1-(3-trifluorométhylbenzyl)cyclohexanol et le chlorhydrate correspondant
et/ou
le 2-(diméthylaminophénylméthyl)-1-(3-trifluorométhylbenzyl)cyclohexanol et/ou le chlorhydrate correspondant.
